# EUROPEAN PATENT APPLICATION

(11) **EP 1 149 805 A1**
(43) Date of publication of application: **31.10.2001**
(21) Application number: 01108228.6
(22) Date of filing: 31.03.2001
(51) Int. Cl.: C02F 11/12, C02F 11/04

(54) **Process for generation of energy from industrially processed fruits and vegetable waste**

(30) Priority: 28.04.2000 DE 10020832
(71) Applicant: Rammensee, Werner, Prof. Dr., 55131 Mainz (DE)
(72) Inventor: Rammensee, Werner, 55131 Mainz (DE); Agiorgitis, George, 64285 Darmstadt (DE)
(74) Representative: Helbing, Jörg, Dr. Dipl.-Chem.

(57) **Abstract**

The present invention provides process for treatment of fruits and vegetable waste from farming and food processing industry, said waste being available only in a particular season and being subjectable to anaerobic fermentation, the process comprises pre treating the biomass waste or a part thereof by dehydration. When the process is used for the anaerobic digestion treatment of citrus peel and rags, the process may further comprise a special deoiling step prior to the anaerobic fermentation.

## Description

The present invention relates to a process for treatment of fruits and vegetable waste from farming and food processing industry, said waste being available only in a particular season and being subjectable to anaerobic fermentation, the process comprises pre-treating the vegetable waste or a part thereof by dehydration. When the process is used for the anaerobic digestion treatment of citrus fruit waste, the process may further comprise a deoiling step prior to the anaerobic fermentation.

In particular, the present invention provides an environmentally-friendly pretreatment system "AGRA" which allows a continuous anaerobic digestion of liquid and solid waste from industrial processing agricultural products, especially citrus, apricots, peaches, tomato, sugar beets waste and their by-products. The pretreated waste can be subjected to anaerobic fermentation as an aqueous suspension singly or in admixture with other biological waste (also of animal origin).

It is established in the prior art that most kinds of organic waste can be subjected to anaerobic fermentation (see, e.g., DE-A-198 05 045, DE-A-198 40 691 and DE-C-36 15 873). However, this technology requires that fermentation can be operated permanently throughout the year since the starting of a fermentation (biogas) reactor until the operation is stable may take several weeks.

It was therefore an object to establish a process which allows a continuous fermentation of organic waste. Moreover, for treatment of biomass waste from agricultural processing industry, said waste being available only in particular seasons and being subjectable to anaerobic fermentation, the process comprises pretreating the biological waste or a part thereof by dehydration.

It is well known to the artesian that the fermentation process of waste of citrus fruits is inhibited by volatile and non-volatile oil components of the citrus fruits. Thus it was a further object of the present invention to make citrus fruit waste accessible for a fermentation treatment.

### Summary of the Invention

It was found that if the organic waste is dehydrated prior to the anaerobic fermentation reaction, the waste can be temporarily stored in Nitrogen innertisate silos after appropriate to ensure permanent operation throughout the year. Moreover, it was found that for citrus fruit waste a deoiling step prior to the anaerobic fermentation also makes said waste accessible to fermentation. The present invention thus provides
(1) a process for treatment of biomass waste from farming and food processing industry, said waste being available only in a particular crop season and being subjectable to anaerobic fermentation, the process comprises pretreating the byproducts waste or a part thereof by dehydration or drying; and
(2) a process for the treatment of citrus fruit waste, in particular as defined in (1) above, which process comprises a deoiling step prior to the anaerobic fermentation.

### Description of the Figure

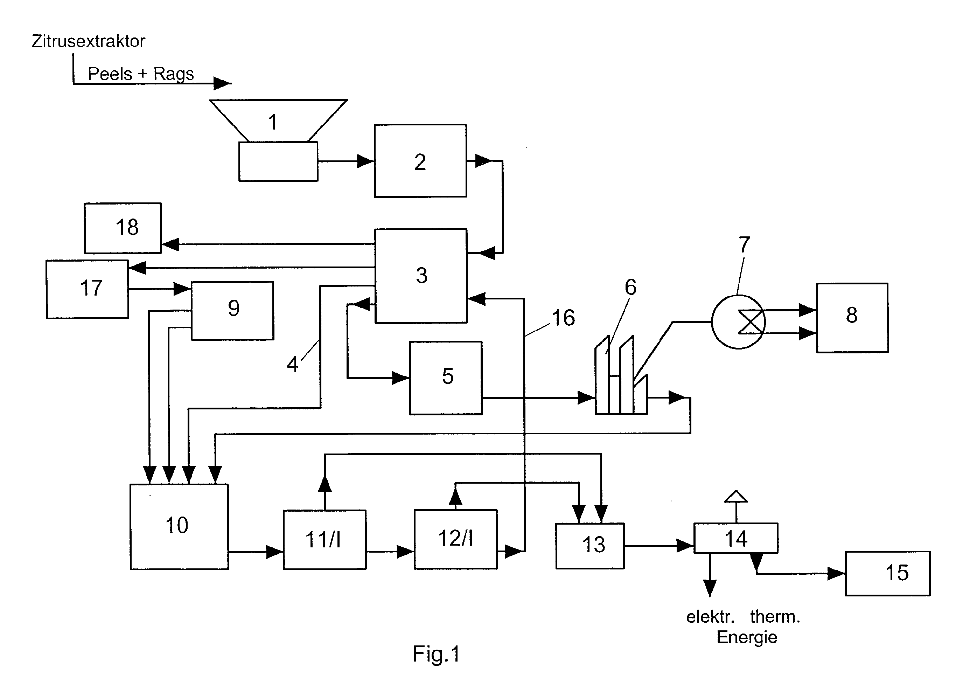

**Figure 1** shows an example of a plant for the fermentation of citrus fruit waste, wherein the symbols have the following meanings:

| | | | |
|---|---|---|---|
| 1 | mill, homogenizer | 11 | fermenter I |
| 2 | buffer tank | 12 | fermenter II |
| 3 | decanter | 13 | biogas tank |
| 4 | semisolid substrate | 14 | block-type thermal power station |
| 5 | molasses buffer tank | 15 | waste heat boiler |
| 6 | evaporator | 16 | fermentation substrate line |
| 7 | d-limonene rectification | 17 | surplus water tank |
| 8 | d-limonene tank | 18 | anaerobic compost silo |
| 9 | surplus water tank | | |
| 10 | buffer and processing tank | | |

### Detailed Description of the Invention

The major byproducts and waste streams generated from citrus processing industries are juice extractor residues such as peel - rags, press liquor from the dewatering of citrus peels prior to drying and the aqueous discharge of the oil-mill centrifuges. These residues peel, pulp and seeds are approx. 50-55% by weight of the fruit processed.

A further problem is represented by the fact, that the fermentation of organic waste which is available only seasonally, for example, obtained only during a relatively short crop duration period, such as citrus or other fruit or vegetables, and in food-processing plants, is difficult to put into practice also because a standstill of the fermentation plant over several months requires a high financial and technical expenditure for the necessary maintenance works during the standstill time and the putting back into operation. In addition, an healthy and efficient operation is extremely doubtful since a standstill of the plant for 6-8 months is no longer feasible. Thus, for example, the harvesting period of citrus fruit in the Mediterranean countries is about from 100 to 150 days, or the sugar beet wastes from 100 to 120 days.

It has been an aspect of the invention to make seasonally available waste last throughout the year and thus to enable a continuous operation of bio-fermentation all year round. To this end, the biological waste must be processed to enable its storage in a silo for at least 6-8 months without fermenting or being otherwise converted biologically. In principle, several methods can be employed for achieving this.
1. Dehydration of the biomass down to a humidity content of about 30 using e.g. a fluidized bed drier. The dehydrated biomass is subsequently stored optionally under N₂ This method is particularly suitable for application to sugar beet waste (cuttings).
2. Dehydration by an evaporator. The liquid biomass is evaporated to about 1/15 of its original volume in an evaporator. The energy is obtained from the waste heat of the block-type thermal power station so that no additional energy needs arise. The dehydrated biomass is subsequently stored optionally under N₂. This method is particularly suitable for citrus fruit waste. Evaporation involves the additional advantage that the concentrated liquid waste is sterilized by this process, and no further measures to prevent fermentation are necessary. In addition, the evaporation removes substances interfering with or inhibiting fermentation. When the process is applied to citrus fruit, water soluble aroma is additionally recovered, which can be used further with profit and thus improves the economic efficiency of the process.
3. The concentrated biomass is stored in a silo optionally flooded with inert gases, like Nitrogen gas. The high N₂ pressure reliably prevents any aerobic or anaerobic fermentation of the biomass.

The application of the appropriate method depends on the respective biomass.

Thus, the total process consists of the following steps:
1. Pretreatment of the biomass waste and effluents: homogenization and mixing, e.g. in a Hammer mill, optionally mixing with other biological waste (cofermentation); adjusting the content of organic dry substance of the aqueous suspension to optimum conditions for fermentation; optionally adjusting the pH value.
2. Dividing the homogenized biomass suspension into a substream for direct fermentation, prior deoiling either mesophilic or thermophilic, and a second substream for dehydration. The ratio of the substreams depends on the distribution and availability of biomass obtained through the seasons.
3. Slurring the biomass concentrate with water in a buffer tank, using the surplus water obtained during dehydration, which can be temporarily stored in water basins.
4. Pumping the biomass into the fermenter.
5. Supplying the biogas, optionally after suitable gas purification and drying, to the block-type thermal power station for the generation of electric power and process steam; recirculating a substream of the process steam (depending on the amount of biomass currently produced) for the drying or evaporating and for controlling the temperature of the fermenters.Citrus fruits are selected from the group consisting of oranges, lemons, limes and grapefruits.

Generally the orange byproducts contain about 2.000 ppm of essence oil crop processed, depending on the juice extraction equipment, with 95% volatile and 5% nonvolatile constituents.

The raw peel oil composition in general consists of terpenes such as lemonene 90 %, 5 % of flavonoids such hexanal, pinene, myrcene, ethylisobutirate and about 5 % of non-volatiles such as wax coating, lipids and pigments.

Investigations have shown that the major problem in the A.D. of citrus peels is the inhibition of fermentation bacteria, resulting from the presence of residual oil components, their toxicity properties is characterized by the appearance of increased levels of benzoic, phenylacetic and phenylpropionic acids.

However, the anaerobic digestion treatment of citrus byproducts cannot be utilised without prior reducing or eliminating the volatile and if possible the non-volatile components into acceptable oil levels for fermentation.

Our investigations using laboratory scale digesters and those in pilot scale with 23m³ working capacity have been performed utilizing several citrus wastes and byproducts such as peel, molasses and pelletized dried peel.

Digestions carried out with waste orange peel obtained from citrus processing plants containing 0.55, 0.45 and/or 0.30 % oil by loading rates of 3 kg_{TS}/m^{3·}d has always shown digestion failure caused by the high oil concentration.

Parallel trials with press liquor (molasse) have shown that a high proportion of oil content is associated with suspended solids, an evaporation of the press liquor did not provide the adequate reduction of all the toxic components due to low evaporation temperature, which causes digestion failure within some days, whereas pretreated feedstock contains less than 0.25 % residual oil, showed less symptoms of toxicity over 120 days of operation. The results obtained clearly indicate that the maximal concentration of oil which can be tolerated in an feedstock with 10 % solids and loadings of up to 3 kg/m³·d represents 0.25 % oil contents which corresponding to a maximal acceptable dosing rate of 0.075g oil/I digestion liquor/d.

The stipulated digestions failure obviously is associated with residual peel oil, characterized by the appearance of aromatic acids, rising volatile fatty acid levels, falling pH, falling methane flow and rising levels of CO₂. These symptoms be often appeared 10 to 20 days after feeding with orange peels had commenced.

Furthermore it can be assured that the presence of aromatic acids in digestion liquors cannot serve as an indication of toxicity or overloading.

Treatments carried out with deoiled feedstocks (up to 0.20 % ) and loads of up to 5.0 kg/m^{3·}d have shown fast biodegradability up to 93.2 % (hydrolysis stage about 60 %), digestion stability and high biogas production (min. 55 % methane and 45 % carbon dioxide) the biogas composition was consistent with that expected from carbohydrate feedstock. Only dried feedstock with total extracted oil shown better fermentation out come. Pelletized orange peel used as feedstock in extensive trials yielded 57 l/kg TS of biogas at loadings of up to 5.8 kg/m³. This is the highest loading rate which has been observed.

The anaerobic digestion results obtained have demonstrated that the best way to convert processed citrus peels and rags into valuable biogas, is the necessity of an efficient oil extraction prior the anaerobic digestion.

Throughout the peel deoiling pretreatment several operational benefits are realizable such as digestion performance, process efficiency, valuable lemonene and water soluble essence production.

Finally, it is advisable to reduce the oil levels in peels prior digestion in to non -toxic levels.

Furthermore, however in order to maintain healthy digestions it is advisable to optimize the levels of nutrients with (NH₄)₂ HPO₄ (1.5-2 g/I) adding to the feedstock. The cost of supplementing a full-scale rector with nutrients have to be established during the commercial adoption of the process. An economic alternative would be to use pig manure as supplement. It is estimated that 4 kg dry matter/m³ of manure each week would maintain N₂ - levels at 200 mg/l which is adequate to ensure efficient and complete digestion of citrus peel wastes.

However, for the digestion reactor design, it is recommendable that the feeding loading rates shall be lower than the observed one such as 4.5 kg TS/m^{3·}d which would be a realistic rate for commercial operations.
Therefore the developed processing system is reliable only prior deoiling of peel and can be performed in two separate treatment ways depending on the peel volume and the industrial waste production, both treatment possibilities provide the maximum of performance and efficiency with respect to environmental protection regulations.

The citrus-byproducts and residues are blend to a ratio of 1:0.5 with processing waste water from the citrus plant prior to milling process. Subsequently the milled peel is pumped to deoiling plant.

Commonly the citrus-processing plants operate aprox. 150 days annually, the generated waste volume is from plant to plant variable.

However, the wastes delivered from citrus processing industries during the seasonal operation are partly stored and treated over a time period of 340 days.

The humidity content of peels and rags is up to 82% the organic solid matter about 18%. The citrus peels consists mainly of pectin, polysaccharide and less content of hemi-cellulose.

| **Operation performance of citrus peel wastes treatment plant** | |
|---|---|
| Loading rate _{OTS} | 4.30 kg / m³.d |
| Digestion time | 16 d |
| Degradability _{TS} | 87 % |
| Degradability _{VS} | 93 % |
| | |
| Plant operation | 350 d/a |
| | |
| Daily plant operation | 24 h |
| Degradation rate | 87 % |
| Expected biogas yield | 0.550 Nm³/kg |
| | |
| Biogas spec.energy content | 5.9 KWh/m³ |
| | |
| Expected essence production | 3 kg/t_{TS} |
| Expected compost production | 10 % |

The prepared feedstock suspension is treated in the digestion reactors under anaerobic mesophilic conditions of 36-38 °C. During the industrial non citrus processing period, the treatment plant operations continuously fed from the stored and deoiled citrus (peel, dried peel or molasse) and from additional fruit effluents generated in summer period.
The expected biogas with a energetic value of 5.9 KWhₜₕ is loaded into co-generators producing electrical and thermal energy. The residual digestate of approx. 10% remaining in the reactor is separated and matured through aerobic treatment to an excellent biocompost.

The innovative pretreatment of citrus-fruits byproducts, shall focused the steps deoiling, separation and feedstock storage.
A further pretreatment system, which is similar to the system as described before, consists of the following subsystems:
The below described treatment has been designed to process efficiently on two different ways orange peels over the annual crop duration period. In case the crop duration for processing plants is limited say 120 days by same waste quantities, than the peel pre-treatment differs due to the peel separation in an solid and in an liquid stage (as is reported) by following evaporation.

The received weighted and subsequent homogenized peels undergoes a necessary pretreatment in order to extract and separate the residual essential oil contents. This is an essential intoxication treatment of citrus waste, because the oil-contents effects definitely to fermentation bacteria activity.

The so deoiled feedstock slurry are diluted with tempered waste water in order to prepare a feedstock (10 % solids) and an alkalinity of pH 6.8, which finally is pumped to the digestion reactor.

The retention time in two stage or even in single stage reactors with a calculated volume of 4.000 m³ (loading rate 4.3 kg/m^{3·}d) is considered to be about 4 hours for hydrolysis and about 16 days for methanisation at 36.5 C° mesophilic conditions.

Another pretreatment concept starts with the peels and rags maceration (9 mm) followed by waste water dilution contains chemicals. The peel slurry follows via pressing an separation process in to a solid and liquid state stage. The separated solid stage upon feedstock preparation undergoes an deoiling treatment and finally is pumped to digestion reactor. The liquid phases are concentrated through evaporation to molasse and stored for a later deoiling and digestion treatment.

The generated biogas yields are calculated in particular case for seed less citrus/fruits on the basis of their organic solids degradation min. 85 % which is expected to be 0.500-0.580 Nm³/kg_{input of TS}, contains about 55 % CH₄ with an energetic value of 5.9 KWh/Nm³.

The digested sludge leaves the fermentation should be dewatered by means of screw presses or belt-filter, subsequently the dewatered sludge material which is a raw compost will be transported via conveyor belt for further aerobic treatment. The separated residual liquor contains active bacteria populations is partly pumped back to fermentation process, the main volume of the waste water is pumped to water treatment plant or can be successfully used for citrus plants watering.

The biogas generated by the fermentation process is stored in a low pressure gas-tank and then is transferred to the cogeneration plant, in case the storage capacity has reached a maximum the biogas than is flared off.

## Claims

1. A process for treatment of biomass waste from farming and food processing industry, said waste being available only in a particular season and being subjectable to anaerobic fermentation, the process comprises pretreating the biological waste or a part thereof by dehydration.

2. Process according to claim 1, wherein said waste is derived from fruits and vegetables, preferably is selected from citrus fruit, tomatoes, sugar beet waste, biological waste of animal origin and organic waste similar to the above mentioned materials.

3. The process according to claim 1, wherein said dehydration is effected by an evaporator operated with the waste heat from the block-type thermal power station, preferably an additional dehydration is effected by drying using a special developed low temperature fluidized bed drier, whereas the drier uses the process heat from the block-type thermal power station.

4. The process according to any of claims 1 to 3, wherein after the pretreatment the waste can be temporarily stored in silos after appropriate to ensure permanent operation throughout the year, preferably the waste shall concentrated or dried in accordance with claim 3 is flooded in a silo with N₂ gas.

5. The process according to any of claims 1 to 4, wherein the anaerobic fermentation of the dehydrated waste is performed in an aqueous suspension and/or singly or in admixture with other dehydrated or non-dehydrated biological waste, which can also be of animal origin.

6. The process according to claim 5, **characterized in that** the concentrated or dried (dehydrated) waste is diluted to an optimum content of organic dry substance using water produced during the dehydration as described in claim 3 and temporarily stored in storage basins, and the diluted waste is again homogenized during this process.

7. The process according to any of claims 5 or 6, **characterized in that**
i) the level of nutrients is optimized by addition of (NH₄)₂ HPO₄ to the feedstock.
ii) the level of nutrients is optimized by addition of pig manure as supplement to the feedstock to maintain preferably a cofermentation.

8. The process according to any of the claims 5 to 7, wherein the waste is sugar beet waste of dried stage is diluted to an optimum content of organic dry substance using water produced during the dehydration or dewatering as described in claim 3.

9. The process according to any of the claims 5 to 8, wherein the anaerobic fermentation provides a methane-containing biogas which can be further utilized for the generation of energy.

10. A process for the treatment of citrus fruit waste, in particular as defined in claims 1 to 9, which process comprises a deoiling step prior to the anaerobic fermentation and/or a dehydration step utilising an vibrating fluidized bed drier even prior deoiling
